# EUROPÄISCHE PATENTSCHRIFT

(11) **EP 3 838 158 B1**
(45) Veröffentlichungstag und Bekanntmachung des Hinweises auf die Patenterteilung: **22.11.2023**
(21) Anmeldenummer: 21151196.9
(22) Anmeldetag: 17.08.2018
(51) Int. Cl.: A61B 6/03, A61B 6/04, A61B 6/00

(54) **COMPUTERTOMOGRAPHIEVORRICHTUNG**
COMPUTED TOMOGRAPHY APPARATUS
DISPOSITIF DE TOMOGRAPHIE ASSISTÉE PAR ORDINATEUR

(30) Priorität: 17.08.2017 DE 102017007722
(43) Veröffentlichungstag der Anmeldung: 23.06.2021
(62) Teilanmeldung aus: 18759896.6
(73) Patentinhaber: Schulze, Thorben, 12165 Berlin (DE)
(72) Erfinder: Schulze, Thorben, 12165 Berlin (DE)
(74) Vertreter: Eisenführ Speiser

(56) Entgegenhaltungen:
- EP-A1- 0 387 956
- US-A1- 2016 128 653
- Jimmy Saunders Alastair Nelson Katrien Vanderperren: "Particularities of Equine CT" In: Eds T. Schwarz and J. Saunders: "Veterinary Computed Tomography", 23. August 2013 (2013-08-23), John Wiley & Sons, XP002786643, Seiten 421-426, DOI: 10.1002/9781118785676.ch39, * Seite 421 - Seite 423; Abbildungen *

## Beschreibung

Die Erfindung betrifft eine Computertomographievorrichtung zum Untersuchen eines Körperteils eines Großtieres und ein Verfahren zum Untersuchen eines Körperteils eines Großtieres mit einer Computertomographievorrichtung.

Ein Computertomographie (CT)-Gerät weist typischerweise eine als Ringtunnel ausgebildete Gantry auf sowie einen in die mittige Öffnung der ringförmigen Gantry einfahrbaren CT-Tisch, auf dem ein Patient oder ein Untersuchungsobjekt gelegt werden kann. In der Gantry sind eine CT-Röhre und der CT-Röhre gegenüber ein Detektorsystem exzentrisch angeordnet, die sich während einer CT-Untersuchung in dem Ringtunnel drehen. Dabei emittiert die CT-Röhre einen Fächerstrahl aus Röntgenstrahlen in Richtung des Detektorsystems. Wenn ein Patient oder ein Untersuchungsobjekt auf dem CT-Tisch in die mittige Öffnung der ringförmigen Gantry eingefahren ist, durchdringen die Röntgenstrahlen das zu untersuchende Gebiet und werden in ihrer Intensität abhängig von den Absorptionseigenschaften innerhalb des beleuchteten Gebiets abgeschwächt. Wenn sich in dem beleuchteten Gebiet Strukturen mit unterschiedlichen Absorptionseigenschaften befinden, beispielsweise Knochen- und Weichteilgewebe, werden die Röntgenstrahlen in dem beleuchteten Gebiet unterschiedlich stark abgeschwächt. Der Detektor detektiert eine zweidimensionale Projektion des in der Regel dreidimensionalen beleuchteten Objekts, wobei die Projektion eine Intensitätskarte der detektierten Röntgenstrahlen repräsentiert. Durch die Drehung der CT-Röhre und des Detektorsystems können während einer CT-Untersuchung eine Vielzahl Projektionen eines zu untersuchenden Objekts aus verschiedenen Richtungen aufgenommen werden, aus denen dann rechnerisch durch einen Computer die ursprüngliche dreidimensionale Struktur des untersuchten Objekts rekonstruiert werden kann.

Jedoch ist ein eingangs beschriebenes handelsübliches CT-Gerät nur begrenzt geeignet, eine CT-Untersuchung an einem stehenden Großtier durchzuführen. Oftmals kann eine CT-Untersuchung an einem liegenden Großtier nur in Vollnarkose durchgeführt werden und ist deshalb vergleichsweise riskant und aufwendig. CT-Geräte, mit denen eine CT-Untersuchung an einem stehenden Großtier möglich ist, sind oftmals auf die Untersuchung von bestimmten Körperteilen eines Großtieres eingeschränkt. Wenn beispielsweise für eine CT-Untersuchung eine auf Schienen verfahrbare Gantry eines CT-Geräts (engl. *Sliding Gantry*) verwendet wird, können bei einem stehenden Großtier, beispielweise einem Pferd, typischerweise nur der Kopf und der Hals und insbesondere nicht die Gliedmaßen untersucht werden. Bei einer solchen Anordnung einer Gantry auf Schienen kann es erforderlich sein, einen Patienten auf einer Hebebühne stehend auf eine bestimmte Höhe anzuheben, damit ein zu untersuchendes Körperteil in der Öffnung der ringförmigen Gantry zu positionieren. Ein Großtier, beispielsweise ein Pferd, auf einer Hebebühne anzuheben stellt ein erhebliches Risiko für das Großtier dar und erschwert eine CT-Untersuchung an einem Großtier zusätzlich, da beispielsweise ein Großtier in der Regel nur langsam angehoben werden kann, wodurch sich eine Untersuchungszeit deutlich verlängert. Eine Anordnung einer Gantry auf Schienen benötigt zudem im Vergleich zu einem eingangs beschriebenen CT-Gerät wesentlich mehr Platz. Beide eingangs beschriebenen Varianten weisen also eine Reihe von Nachteilen auf.

In US 9,301,726 B2 wird eine CT-Maschine zum Scannen eines stationären Patienten, beispielsweise einen Menschen oder ein Pferd, beschrieben. Die CT-Maschine weist Gelenkarme mit zwei Gelenkstangen auf, um eine CT-Gantry auf einer beliebigen Trajektorie zu bewegen und unterscheidet sich somit deutlich von dem grundlegenden Aufbau eines eingangs beschriebenen, handelsüblichen CT-Geräts.

US 2016/128653 A1 beschreibt, wie eine auf einem Rollwagen angeordnete CT-Röhre für beispielsweise die Untersuchung eines Pferdekopfes genutzt werden kann. Die CT-Röhre ist dabei auf dem Rollwagen verfahrbar. Auch EP 0 387 956 A1 beschreibt eine CT-Röhre, die auf einem Rollwagen angeordnet ist und die um eine horizontale Achse schwenkbar ist. Jimmy Saunders, Alastair Nelson und Katrien Vanderperren beschreiben in "Particularities of Equine CT" den Gebrauch von Computertomographen für die Untersuchung von Pferden.

Es ist eine Aufgabe der Erfindung eine verbesserte Computertomographievorrichtung zum Untersuchen eines Körperteils eines stationär stehenden Großtieres sowie ein verbessertes Verfahren zum Untersuchen eines Körperteils eines stationär stehenden Großtieres mit einer Computertomographievorrichtung bereitzustellen.

Hinsichtlich der Computertomographievorrichtung wird die Aufgabe der Erfindung gelöst durch eine Computertomographievorrichtung zum Untersuchen eines Körperteils eines Großtieres aufweisend ein Computertomographie (CT)-Gerät und eine Plattform, auf der das CT-Gerät montiert ist. Das CT-Gerät umfasst eine ringförmige Gantry und einen CT-Tisch zur Aufnahme eines zu untersuchenden Körperteils eines Großtieres. Die Gantry ist relativ zu der Plattform auf der Plattform angeordnet.

Der CT-Tisch ist relativ zu der Plattform und zu der Gantry horizontal beweglich mit der Gantry und/oder der Plattform derart verbunden, sodass der CT-Tisch in eine mittige Öffnung der ringförmigen Gantry eingefahren werden kann.

Erfindungsgemäß umfasst die Computertomographievorrichtung weiterhin eine Verschiebevorrichtung, die mit der Plattform verbunden und ausgebildet ist, die Plattform mit dem montierten CT-Gerät relativ zu einer Standfläche eines Großtieres horizontal zu verschieben. Die Verschiebevorrichtung ist weiterhin ausgebildet, in einem Betriebszustand die Plattform mit dem montierten CT-Gerät derart zu verschieben, dass der CT-Tisch während einer Relativbewegung zur Gantry relativ zu der Standfläche stationär bleibt.

Im Rahmen dieser Beschreibung wird unter einer Standfläche ein fester Untergrund verstanden, der sich auf derjenigen Seite der Gantry befindet, die dem CT-Tisch gegenüberliegt. Die Standfläche befindet sich außerhalb der Computertomographievorrichtung. Während einer CT-Untersuchung mit der erfindungsgemäßen Computertomographievorrichtung steht ein zu untersuchendes Großtier stationär auf der Standfläche.

Das CT-Gerät der erfindungsgemäßen Computertomographievorrichtung ist wie ein handelsübliches CT-Gerät aufgebaut und umfasst eine Gantry und einen CT-Tisch zur Aufnahme eines zu untersuchenden Körperteils eines Großtieres. Für eine CT-Untersuchung kann der CT-Tisch in die Gantry eingefahren werden, um so wie eingangs beschrieben Projektionen aus verschiedenen Richtung aufzunehmen, die computergestützt verarbeitet werden können. Vorteilhafterweise muss also an dem CT-Gerät der erfindungsgemäßen Computertomographievorrichtung keine wesentliche Veränderung im Vergleich zu einem handelsüblichen CT-Gerät vorgenommen werden.

Der Erfindung liegt nun die Erkenntnis zugrunde, dass es vorteilhaft ist, ein handelsübliches CT-Gerät zu verwenden, da sich so der Aufwand eines Umbaus oder der Aufwand einer Neuentwicklung eines CT-Geräts und dadurch entstehende Kosten einsparen lassen. Ein weiterer Vorteil eines handelsüblichen CT-Geräts liegt darin, dass die Computertomographievorrichtung nicht auf ein bestimmtes CT-Gerät festgelegt ist, sondern mit einer Vielzahl handelsüblicher CT-Geräte, und insbesondere herstellerunabhängig, realisiert werden kann.

Die Erfindung beruht auf der Überlegung, dass ein handelsübliches CT-Gerät an sich nicht geeignet ist, ein Körperteil eines auf einer Standfläche stationär stehenden Großtieres zu untersuchen, da das zu untersuchende Körperteil des Großtieres für die Untersuchung auf dem CT-Tisch durch Gantry fahren muss. Ein Großtier müsste dann seine stationäre Position auf der Standfläche aufgeben, um der Bewegung des einfahrenden CT-Tisches zu folgen. Dadurch würde eine CT-Untersuchung eines Körperteils deutlich erschwert und das Großtier würde zusätzlichem Stress ausgesetzt werden.

Jedoch haben die Erfinder erkannt, dass dieser Nachteil aufgelöst werden kann, wenn die Computertomographievorrichtung eine Plattform und eine mit der Plattform verbundene Verschiebevorrichtung umfasst. Erfindungsgemäß ist das CT-Gerät deshalb auf der Plattform montiert. Die Plattform dient also als Stellfläche für das CT-Gerät und ist ausgebildet ein CT-Gerät zu tragen.

Die Verschiebevorrichtung ist ausgebildet, die Plattform relativ zu der Standfläche eines Großtieres horizontal zu verschieben. Die Standfläche des Großtieres liegt außerhalb der Plattform, sodass sich ein stationär stehendes Großtier selbst nicht bewegt, wenn sich die Plattform mit dem CT-Gerät relativ zu der Standfläche horizontal verschiebt. Während einer CT-Untersuchung liegt jedoch das zu untersuchende Körperteil des Großtieres auf dem CT-Tisch auf und würde mit dem CT-Tisch in die Gantry einfahren. Um ein horizontales Verschieben des zu untersuchenden Körperteils während einer CT-Untersuchung relativ zu der Standfläche, also auch relativ zu dem übrigen Großtier, zu verhindern, ist erfindungsgemäß vorgesehen, dass die Computertomographievorrichtung wenigstens einen Betriebszustand aufweist, in dem die Plattform derart horizontal verschoben wird, dass der CT-Tisch während einer Relativbewegung zur Gantry relativ zu der Standfläche stationär bleibt.

In diesem Betriebszustand der Computertomographievorrichtung steht der CT-Tisch für einen außenstehenden Beobachter still. Für einen außenstehenden Beobachter ergibt sich also ein Bild, als wenn die Gantry, die an sich unbeweglich auf der Plattform montiert ist, über den CT-Tisch und insbesondere über ein zu untersuchendes Körperteil eines Großtieres hinwegfahren würde. In diesem Betriebszustand bewegen sich für einen außenstehenden Beobachter gerade diejenigen Elemente des CT-Geräts, die sich bei einer handelsüblichen Verwendung ohne Plattform und Verschiebevorrichtung nicht bewegen würden. Vorteilhafterweise steht in diesem Betriebszustand also der CT-Tisch für einen außenstehenden Beobachter still, während sich die Gantry über den CT-Tisch und ein zu untersuchender Körperteil eines Großtieres hinwegzubewegen scheint. Durch die Computertomographievorrichtung muss ein stationär stehendes Großtier während einer CT-Untersuchung also nicht seine Position auf der Standfläche aufgeben und bleibt somit unbewegt in Bezug auf das CT-Gerät. Alle für die CT-Untersuchung erforderlichen Bewegungen führt die Computertomographievorrichtung aus.

Ein Großtier steht während einer CT-Untersuchung also unbeweglich auf festem Grund. Vorteilhafterweise bewegt sich ein Großtier dann während einer CT-Untersuchung nicht und insbesondere auch nicht das zu untersuchende Körperteil des Großtieres. Eine CT-Untersuchung kann also besonders zuverlässig an einem stationär stehenden Großtier durchgeführt werden, ohne dass das Großtier besonders narkotisiert werden muss und ohne, dass das Großtier zusätzlichem Stress ausgesetzt wird.

Im Folgenden werden bevorzugte Weiterbildungen der erfindungsgemäßen Computertomographievorrichtung beschrieben.

Vorzugsweise werden der CT-Tisch und die Plattform während einer CT-Untersuchung mit vom Betrag her gleicher Geschwindigkeit in jeweils zueinander entgegengesetzte Richtungen verschoben. Dadurch wird die Bewegung des in die Gantry einfahrenden CT-Tisches durch die entgegengesetzt gerichtete Bewegung der Plattform für einen außenstehenden Beobachter kompensiert. Für einen außenstehenden Beobachter steht dann der CT-Tisch still während sich die Gantry über den CT-Tisch hinwegzubewegen scheint. In anderen Worten bewegen sich insbesondere der CT-Tisch und die Plattform derart, dass die Bewegung des CT-Tisches relativ zur Gantry und zur Plattform durch eine Relativbewegung der Plattform zu Standfläche in die dazu entgegengesetzte Richtung, für einen außenstehenden Beobachter und insbesondere für ein auf der Standfläche befindliches Großtier kompensiert wird. Dadurch steht der CT-Tisch für einen außenstehenden Beobachter still, während die Gantry, die an sich unbeweglich auf der Plattform montiert ist, für den außenstehenden Beobachter über den CT-Tisch und insbesondere über ein zu untersuchendes Körperteil eines Großtieres hinwegzufahren scheint.

In einer besonders bevorzugten Weiterbildung weist die Computertomographievorrichtung zusätzlich eine Hebevorrichtung auf. Die Hebevorrichtung ist ausgebildet, die Plattform relativ zu der Standfläche eines Großtieres anzuheben oder abzusenken. Das auf der Plattform montierte CT-Gerät kann mittels der Hebevorrichtung und der Verschiebevorrichtung also relativ zu der Standfläche sowohl vertikal angehoben und abgesenkt als auch horizontal verschoben werden.

Durch eine Hebevorrichtung können vorteilhafterweise mehrere Körperteile eines Großtieres in unterschiedlichen Höhen nacheinander untersucht werden, ohne dass das Großtier seine Position auf der Standfläche wesentlich ändern muss. Zum Beispiel können nacheinander der Kopf, der Hals und die Vorderbeine eines Großtieres untersucht werden. Vorzugsweise ist die Hebevorrichtung ausgebildet, das CT-Gerät in einem Höhenbereich von ein bis zwei Metern anzuheben und abzusenken, sodass beispielsweise die Schulterhöhe eines Pferdes von dem Hubbereich des CT-Geräts umfasst ist. Vorteilhafterweise kann ein Großtier unbeweglich auf festem Grund vor der Computertomographievorrichtung stehen. Besonders vorteilhaft kann also auf eine Hebebühne zum Anheben eines Großtiers auf eine für eine CT-Untersuchung notwendige Höhe verzichtet werden.

Alle nötigen Bewegungen zum Durchführen einer CT-Untersuchung eines oder nacheinander mehrerer Körperteile eines Großtieres werden allein von der Computertomographievorrichtung ausgeführt.

Bevorzugt umfasst die Verschiebevorrichtung ein Schienensystem, welches auf einer Bodenplatte angeordnet ist. Vorzugsweise ist die Plattform mit dem montierten CT-Gerät auf dem Schienensystem angeordnet, sodass die Bodenplatte und die Plattform durch das Schienensystem miteinander verbunden und relativ zueinander beweglich sind. Die Bodenplatte hat vorzugsweise dieselbe Länge und Breite wir die Plattform. Der benötigte Platz zum Installieren der Computertomographievorrichtung erhöht sich also nicht durch die Bodenplatte. Die Bodenplatte kann auch nicht vollflächig, sondern als Rahmen ausgebildet sein. Die Bodenplatte ist ausgebildet, das Gewicht der Plattform und des darauf montierten CT-Geräts zu tragen. Durch das Schienensystem können die Bodenplatte und die Plattform vergleichsweise reibungsarm in eine definierte Richtung zueinander verschoben werden.

Bevorzugt umfasst die Verschiebevorrichtung einen Verschiebemotor, der ausgebildet ist, einen Verschiebeantrieb zum Verschieben der Plattform relativ zu der Standfläche eines Großtieres bereitzustellen. Vorteilhafterweise kann die Verschiebegeschwindigkeit der Plattform bis zu 14 cm pro Sekunde betragen. Der Verschiebemotor kann also vergleichsweise leistungsstark sein. Der Verschiebemotor ist vorzugsweise ein Elektromotor. Der Verschiebemotor ist insbesondere ausgebildet eine Leistung bereitzustellen, die ausreicht, die über ein Schienensystem miteinander verbundene Plattform und Bodenplatte gegeneinander zu verschieben.

In einer Weiterbildung umfasst die Verschiebevorrichtung eine Gewindespindel und ein auf der Gewindespindel verschiebbar gelagertes Gegengewinde. Bevorzugt ist der Verschiebemotor zum Antreiben der Gewindespindel beispielsweise über einen Riemen mit der Gewindespindel verbunden, und das Gegengewinde an der Plattform angeordnet, sodass im Betrieb des Verschiebemotors eine Rotation der Gewindespindel in eine translatorische Verschiebung der Plattform relativ zu der Standfläche umgewandelt wird. Durch die Gewindespindel und dem darauf verschiebbar, aber nicht drehbar gelagerten Gegengewindes kann eine Drehbewegung des Verschiebemotors, der die Gewindespindel antreibt, in eine relative Verschiebebewegung des Gegengewindes und somit der Plattform gegenüber der Gewindespindel umgesetzt werden. Es handelt sich hierbei also um einen Spindelantrieb.

Vorzugsweise umfasst das CT-Gerät einen CT-Motor zum motorbetriebenen Einfahren des CT-Tisches in die Gantry.

In einer Weiterbildung umfasst das CT-Gerät einen CT-Antrieb, insbesondere mit einem CT-Motor, zum motorbetriebenen Einfahren des CT-Tisches in die Gantry und die Verschiebevorrichtung einen Verschiebemotor umfasst. Bevorzugt sind dann der Verschiebeantrieb und der CT-Antrieb durch eine Kopplung mechanisch miteinander gekoppelt.

In einer Weiterbildung ist die Kopplung derart ausgebildet, dass ein CT-Antrieb direkt durch eine mechanische Kopplung abgegriffen und an den Verschiebeantrieb übertragen wird. Eine mechanische Kopplung kann beispielsweise durch eine Spindel realisiert sein, über die der CT-Antrieb und der Verschiebeantrieb miteinander verbunden sind.

In einer Weiterbildung sind der Verschiebeantrieb und der CT-Antrieb derart miteinander gekoppelt, dass sich der CT-Tisch und die Plattform im Betrieb mit vom Betrag her gleicher Geschwindigkeit in jeweils zueinander entgegengesetzte Richtungen verschieben. Vorzugsweise fährt der CT-Tisch in die Öffnung der Gantry ein während die Plattform in die entgegengesetzte Richtung verschoben wird. Beide Bewegungen werden also vorzugsweise zeitgleich ausgeführt. Dadurch, dass sich der CT-Tisch und die Plattform während einer CT-Untersuchung mit vom Betrag her gleicher Geschwindigkeit in zueinander entgegengesetzte Richtung bewegen, wird die Bewegung des CT-Tisches für einen außenstehenden Betrachter durch die Bewegung der Plattform kompensiert, sodass der CT-Tisch für einen außenstehenden Betrachter während einer CT-Untersuchung ortsfest ist.

In einer Weiterbildung ist die Kopplung des CT-Antriebs und des Verschiebeantriebs derart gestaltet, dass ein Startsignal am CT-Motor abgegriffen und an den Verschiebemotor übertragen wird. Die Plattform wird dann vorzugsweise durch den Verschiebemotor aufgrund des empfangenen Startsignals in eine Richtung verschoben, die der Richtung in die der CT-Tisch in die Öffnung der ringförmigen Gantry einfährt entgegengesetzt ist.

Beispielweise können eine Anzahl unterschiedlicher Geschwindigkeiten vorzugsweise in ein Steuergerät des Verschiebemotors einprogrammiert sein. Für eine CT-Untersuchung kann dann ein CT-Protokoll ausgewählt werden, in welchem der CT-Tisch entsprechend einer der einprogrammierten Geschwindigkeiten des Verschiebemotors mit dieser Geschwindigkeit in die Gantry eingefahren wird. Das Startsignal des CT-Motors kann dann abgegriffen und an den Verschiebemotor übertragen werden, sodass sich der CT-Tisch und die Plattform im Wesentlichen zeitgleich mit vom Betrag her gleicher Geschwindigkeit in zueinander entgegengesetzte Richtungen bewegen. Dadurch steht der CT-Tisch für einen außenstehenden Beobachter still, während die Gantry für den außenstehenden Beobachter über den CT-Tisch hinweg fährt.

In einer Weiterbildung ist die Kopplung des CT-Antriebs und des Verschiebeantriebs derart gestaltet, dass die Geschwindigkeit und/oder die Position des CT-Tisches und/oder der Plattform durch einen entsprechenden Geschwindigkeits- oder Positionssensor gemessen wird. Ein Geschwindigkeitssignal und/oder Positionssignal, welche entsprechend eine gemessene Geschwindigkeit oder Position repräsentieren, kann dann an den CT-Motor und/oder den Verschiebemotor als Stellgröße übertragen und verwendet werden, den CT-Motor und/oder den Verschiebemotor zu regeln. Geschwindigkeit und/oder Position stellen dann Stellgrößen dar, mit denen der CT-Motor und/oder der Verschiebemotor, beispielsweise die Drehzahl als Regelgröße, gezielt beeinflusst werden kann. Insbesondere werden die Stellgrößen verwendet den CT-Motor und/oder den Verschiebemotor derart zu regeln, dass sich CT-Tisch und Plattform mit vom Betrag her gleicher Geschwindigkeit in zueinander entgegensetzte Richtungen bewegen. Auch dadurch kann eine Synchronisation der Bewegungen des CT-Tisches und der Plattform derart erreicht werden, dass der CT-Tisch für einen außenstehenden Beobachter stillsteht, während die Gantry über den CT-Tisch hinwegzufahren scheint.

In einer Weiterbildung, in der die Plattform mit dem CT-Gerät auf einer Bodenplatte angeordnet ist, umfasst die Hebevorrichtung eine Mehrzahl Gewindespindeln. Die Gewindespindeln der Hebevorrichtung sind vorzugsweise in Bezug auf die Bodenplatte senkrecht in Richtung der Plattform seitlich an der Bodenplatte angeordnet und jeweils über ein verschiebbar gelagertes Gegengewinde mit der Bodenplatte verbunden. An der Bodenplatte sind also bevorzugt Gegengewinde angebracht, die jeweils verschiebbar auf einer Gewindespindel gelagert sind. Wenn die Gewindespindeln angetrieben werden, kann so die Bodenplatte und die auf der Bodenplatte angeordnete Plattform mit dem CT-Gerät angehobenen oder abgesenkt werden.

Vorzugsweise sind vier Säulen vorgesehen, in denen jeweils eine Gewindespindel angeordnet ist. Die vier Säulen sind bevorzugt seitlich an der Bodenplatte angeordnet, sodass die Gewindespindeln in den Säulen jeweils in Bezug auf die Bodenplatte senkrecht in Richtung der Plattform verlaufen. Die Säulen tragen zur Stabilität der Hebevorrichtung bei und schützen die Gewindespindeln der Hebevorrichtung vor äußeren Einflüssen, beispielsweise Verschmutzung.

In einer Weiterbildung umfasst die Hebevorrichtung wenigstens einen Hebemotor, der zum Antreiben wenigstens einer der Mehrzahl der Gewindespindeln der Hebevorrichtung mit einer der Gewindespindeln verbunden und ausgebildet ist, einen Hebeantrieb zum Anheben oder Absenken der Plattform relativ zu der Standfläche eines Großtieres bereitzustellen. Der Hebemotor ist vorzugsweise ausgebildet, eine Leistung bereitzustellen, die ausreicht eine Gewindespindel anzutreiben und somit die Bodenplatte und die darauf angeordnete Plattform mit CT-Gerät anzuheben oder abzusenken. Auch der Hebemotor kann über Riemen mit einer oder mehreren Gewindespindeln verbunden sein. Der Hebemotor ist vorzugsweise ein Elektromotor.

Vorzugsweise sind wenigstens zwei der Gegengewinde der Hebevorrichtung durch eine Synchronisationsvorrichtung mechanisch miteinander verbunden. Eine Synchronisationsvorrichtung kann beispielsweise durch ein Verbindungselement realisiert sein, welches zwei Gegengewinde mechanisch miteinander koppelt. Ein Verbindungselement kann beispielsweise eine Metallstange oder ein Betonelement sein. Wenn also eine Gewindespindel angetrieben wird und sich das darauf verschiebbar gelagerte Gegenwinde entlang der Gewindespindel verschiebt, verschiebt sich ein über eine Synchronisationsvorrichtung mit dem Gegengewinde verbundenes weiteres Gegengewinde synchron mit dem Gegengewinde der angetriebenen Gewindespindel. Es kann auch vorteilhaft sein, mehr als zwei Gegengewinde über eine Synchronisationsvorrichtung miteinander mechanisch zu verbinden.

In einer Weiterbildung, in der die Plattform mit dem CT-Gerät auf einer Bodenplatte angeordnet ist, umfasst die Hebevorrichtung bevorzugt wenigstens einen hydraulischen Druckzylinder. Der Druckzylinder ist vorzugsweise derart in Bezug auf die Bodenplatte angeordnet, dass die Bodenplatte durch Druckbeaufschlagung des Druckzylinders relativ zu der Standfläche angehoben oder abgesenkt werden kann.

In einer bevorzugten Weiterbildung weist die Computertomographievorrichtung einen Untersuchungsstand auf. Der Untersuchungsstand umfasst vorzugsweise eine Haltevorrichtung und eine von der Haltevorrichtung gehaltene Ablage auf der ein Körperteil eines Großtieres abgelegt werden kann. Der Untersuchungstand ist bevorzugt derart angeordnet, dass sich wenigstens die Ablage während eines Verschiebens der Plattform durch die Verschiebevorrichtung nicht mitbewegt wird. Vorzugsweise ist die Ablage derart ausgebildet, dass sie in einer Höhe in Bezug auf das CT-Gerät verstellt werden kann. Es ist denkbar, dass die Ablage derart an der Haltevorrichtung angeordnet ist, dass die Ablage von der Haltevorrichtung abgenommen und in einer unterschiedlichen Höhe wieder an der Haltevorrichtung angebracht werden kann. Beispielsweise kann die Ablage durch eine Schraubverbindung an der Haltevorrichtung angebracht sein und die Haltevorrichtung entlang der Höhe Löcher zum Befestigen der Ablage durch die Schraubverbindung aufweisen. Die Höhe der Ablage in Bezug auf ein zu untersuchendes Großtier kann also vorteilhafterweise entsprechend der Höhe des Großtieres an das CT-Gerät angepasst werden. Vorzugsweise ist die Ablage derart angeordnet und auf eine Höhe eingestellt, dass die Ablage durch ein Verschieben der Plattform mit dem CT-Gerät in die Öffnung der ringförmigen Gantry eingefahren werden kann, insbesondere soweit, dass die Ablage durch die Öffnung der ringförmigen Gantry hindurch ragt. Die Gantry kann dann über die Ablage hinweg fahren, sodass sich die Ablage während einer CT-Untersuchung wenigstens mit einem Teilbereich innerhalb der Öffnung der ringförmigen Gantry befindet.

Vorzugsweise ist Ablage und insbesondere die Teilbereiche der Ablage, die sich während einer CT-Untersuchung potentiell innerhalb der Gantry befinden können, aus einem Material gebildet, welches transparent für Röntgenstrahlen ist. Auch zum Fixieren eines zu untersuchenden Körperteils auf dem CT-Tisch oder auf der Ablage wird bevorzugt ein Fixierelement, beispielsweise ein Gurt, aus einem röntgentransparenten Material verwendet.

Wenn eine Computertomographievorrichtung eine Bodenplatte umfasst, auf der die Plattform mit dem montierten CT-Gerät angeordnet ist, ist die Haltevorrichtung vorzugsweise an der Bodenplatte angebracht, sodass sich die Haltevorrichtung und die Ablage gemeinsam mit der Bodenplatte bewegen, wenn diese durch eine Hebevorrichtung relativ zu der Standfläche angehoben oder abgesenkt wird.

Dadurch, dass die Haltevorrichtung an der Bodenplatte angebracht ist, ist die Haltevorrichtung mit der Ablage zusammen mit der Bodenplatte höhenverstellbar. Wenn also die Bodenplatte mit einer Hebevorrichtung angehoben oder abgesenkt wird, wird die Haltevorrichtung mit der Ablage entsprechend zusammen mit der Bodenplatte angehoben oder abgesenkt. Die Ablage kann so an die Höhe eines zu untersuchenden Großtieres und insbesondere eines zu untersuchendes Körperteils, beispielsweise an die Höhe eines Pferdekopfes oder Halses, angepasst werden.

Bevorzugt ist die Haltevorrichtung derart ausgebildet, dass sie einen Schutz des CT-Geräts vor einem Einwirken eines zu untersuchenden Großtieres gewährleistet. Beispielsweise kann ein verstellbarer Gurt so an der Haltevorrichtung angebracht sein, dass ein Weg nach vorne in Richtung des CT-Geräts für ein Großtier versperrt ist, um so das CT-Gerät beispielsweise vor einem Tritt zu schützen.

Vorzugsweise ist die Haltevorrichtung derart an der Bodenplatte angeordnet, dass sie leicht abnehmbar ist, beispielsweise durch ein Lösen einer Anzahl von Schrauben oder anderer Befestigungsmittel. Vorteilhafterweise kann dann eine Haltevorrichtung für eine CT-Untersuchung eines Großtieres in Vollnarkose abgenommen und ein OP-Tisch unmittelbar an die Gantry angrenzend positioniert werden.

Da der CT-Tisch während einer CT-Untersuchung relativ zu der Standfläche eines Großtieres stationär bleibt, kann die Ablage auch auf der dem CT-Tisch zugewandten Seite der Ablage mit dem CT-Tisch verbunden sein. Dadurch kann die Stabilität der Ablage erhöht werden. In einer Weiterbildung weist eine Computertomographievorrichtung entsprechend eine Haltevorrichtung mit einer Ablage auf, wobei die Ablage an der dem CT-Tisch zugewandten Seite mit dem CT-Tisch verbunden ist. Durch ein Verschieben der Plattform kann die Ablage dann in die Öffnung der ringförmigen Gantry eingefahren werden.

In einer Weiterbildung ist die Gantry eines handelsüblichen CT-Geräts in Bezug auf den CT-Tisch um 180° gedreht. Vorteilhafterweise befinden sich dann die exzentrisch in der Gantry angeordnete CT-Röhre und der Detektor näher an dem zu untersuchenden Großtier, da sich das Großtier für eine CT-Untersuchung auf der dem CT-Tisch gegenüberliegenden Seite der Gantry befindet und somit auf derjenigen Seite, auf der sich ursprünglich kein zu untersuchender Patient während einer CT-Untersuchung befindet.

In einer Weiterbildung ist der CT-Tisch ausgekoppelt, d.h. der CT-Motor läuft frei. Vorteilhafterweise kann der CT-Tisch dann frei bewegt und auch als Auflagefläche für ein Körperteil eines Großtieres in beliebiger Stellung fixiert werden.

In einer Weiterbildung ist die Ablage auf der dem CT-Tisch zugewandten Seite mit dem CT-Tisch mechanisch verbunden, und der CT-Tisch ausgekoppelt und dadurch in Bezug auf das übrige CT-Gerät frei beweglich, sodass der mit der Ablage verbundene CT-Tisch, wenn das auf der Plattform montierte CT-Gerät zusammen mit der Plattform durch die Verschiebevorrichtung verschoben wird, durch die mechanische Verbindung mit der Ablage relativ zu der Standfläche während einer Relativbewegung zur Gantry stationär bleibt. Wenn also die Plattform durch die Verschiebevorrichtung verschoben wird, bewegt sich das gesamte auf der Plattform montierte CT-Gerät mit der Plattform mit. Die Gantry scheint sich also relativ zu der Standfläche des Großtiers zu verschieben. Der CT-Tisch ist mit der Ablage verbunden, wobei die Ablage so angeordnet ist, dass sie sich nicht mit der Plattform mitbewegt, wenn diese verschoben wird. Die Ablage hält also den CT-Tisch in einer festen Position. Dies ist möglich, da der CT-Tisch ausgekoppelt und dadurch in Bezug auf das übrige CT-Gerät frei beweglich ist. Für einen außenstehenden Beobachter sieht es dann so aus, dass während eines Verschiebens der Plattform der CT-Tisch stillzustehen scheint und die Gantry über den CT-Tisch hinwegzufahren scheint.

Vorzugsweise weist die Gantry einen Laser auf, der in der Gantry derart angeordnet und ausgebildet ist, während einer CT-Untersuchung durch emittiertes Laserlicht das momentan untersuchte Gebiet sichtbar zu machen.

In einer Weiterbildung umfasst die Computertomographievorrichtung Strahlenschutzvorrichtungen, insbesondere Bleimäntel, die jeweils vor und hinterder Gantry angebracht sind. Vorteilhafterweise kann dann Untersuchungspersonal während einer CT-Untersuchung eines Großtieres mitwirken und beispielweise neben einem Pferd ein Pferdebein als auch vor einem Pferd dessen Pferdekopf fixieren ohne einer gesundheitsschädlichen Strahlendosis ausgesetzt zu sein.

Vorher beschriebene Computertomographievorrichtungen können auch innerhalb einer Computertomographie-Untersuchungsanordnung verwendet werden, wobei die Computertomographie-Untersuchungsanordnung eine Computertomographievorrichtung, eine Standfläche für ein Großtier und eine Bodengrube umfasst. Die Computertomographievorrichtung ist in der Bodengrube angeordnet, sodass sich wenigstens ein Teil der Computertomographievorrichtung auf einem im Vergleich zu der Standfläche niedrigeren Niveau befindet. Die Standfläche befindet sich außerhalb der Bodengrube an diejenige Seitenwand der Bodengrube angrenzend, die der Gantry der Computertomographievorrichtung am nächsten ist.

Vorzugsweise ist die Bodengrube so tief in Bezug auf die Standfläche gestaltet, dass sich der CT-Tisch des CT-Geräts, wenn das CT-Gerät durch eine Hebevorrichtung vollständig abgesenkt ist, auf derselben Höhe wie die Standfläche befindet. In diesem Zustand ist es vorteilhafterweise möglich, dass ein Kopf eines Pferdes oberhalb der Gantry positioniert werden kann. Ein Vorderbein des Pferdes kann dann schräg nach vorne auf eine Ablage eines Untersuchungsstandes oder den CT-Tisch gestellt werden, sodass die Gantry während einer CT-Untersuchung relativ zu der Standfläche des Pferdes über das Vorderbein hinwegzufahren scheint. Ein Hinterbein eines Pferdes kann entsprechend von dem Pferd aus schräg nach hinten auf eine Ablage oder den CT-Tisch gestellt werden. Dadurch, dass die Computertomographievorrichtungen vorzugsweise zu 50% in die Bodengrube eingelassen ist, sind besonders vorteilhaft CT-Untersuchungen an den Gliedmaßen eines stehenden Großtieres möglich.

Durch die Hebevorrichtung kann ein CT-Gerät dann vorzugsweise bis zu zwei Meter angehoben werden, sodass im angehobenen Zustand dann auch eine CT-Untersuchung des Kopfes und des Halses eines Großtieres möglich ist. Mit der Computertomographie-Untersuchungsanordnung können also besonders vorteilhaft sowohl die Gliedmaßen als auch der Kopf oder der Hals eines stationär stehenden Großtieres untersucht werden. Insbesondere ermöglicht die Computertomographie-Untersuchungsanordnung, dass eine solche Untersuchung besonders vorteilhaft mit einem handelsüblichen CT-Gerät durchgeführt werden kann.

Wenn sich die Standfläche eines Großtieres auf einem Podest befindet, kann ein Effekt erzielt werden, der dem Anordnen einer Computertomographievorrichtung in einer Bodengrube ähnelt. Das Podest ist dann derart in Bezug auf die Computertomographievorrichtung angeordnet, dass das Pferd an einer identischen Position allerdings erhöht steht. Durch das Podest befindet sich die Standfläche vorzugsweise auf Höhe das CT-Tisches, wenn das CT-Gerät durch eine Hebevorrichtung vollständig abgesenkt ist. In dieser Anordnung lassen sich dann besonders vorteilhaft die Gliedmaßen eines Großtieres mit der Computertomographievorrichtung untersuchen. Durch eine Hebevorrichtung kann das CT-Gerät dann in einem Hubbereich von bis zu zwei Metern angehoben werden, sodass auch der Kopf oder Hals des auf dem Podest stationär stehenden Großtieres vergleichsweise leicht mit der Computertomographievorrichtung untersucht werden können.

Hinsichtlich des Verfahrens wird die eingangs genannte Aufgabe der Erfindung gelöst durch ein Verfahren zum Untersuchen eines Körperteils eines Großtieres mit einer Computertomographievorrichtung. Die Computertomographievorrichtung weist ein Computertomographie (CT)-Gerät und eine Plattform, auf der der CT-Gerät montiert ist, auf. Das CT-Gerät umfasst eine ringförmigen Gantry und einen CT-Tisch zur Aufnahme eines zu untersuchenden Körperteils eines Großtieres. Der CT-Tisch und die Gantry sind zueinander beweglich miteinander verbunden, sodass der CT-Tisch in eine Öffnung der ringförmigen Gantry eingefahren werden kann.

Das erfindungsgemäße Verfahren weist die Schritte auf:
- Positionieren eines Großtieres auf einer Standfläche,
- Auflegen eines zu untersuchenden Körperteils auf den CT-Tisch oder eine Ablage des CT-Geräts,
- Verschieben der Plattform mit dem CT-Gerät relativ zu der Standfläche,
wobei das Verschieben der Plattform in einem Betriebszustand derart gestaltet ist, dass der CT-Tisch oder die Ablage während einer Relativbewegung zur Gantry relativ zu der Standfläche stationär bleiben.

In einer Weiterbildung des erfindungsgemäßen Verfahrens ist vorgesehen, dass in dem Betriebszustand der CT-Tisch in die Öffnung der ringförmigen Gantry in eine erste Richtung einfährt, und die Plattform in eine zweite Richtung, die der ersten Richtung entgegengesetzt ist verschoben wird, wobei das Einfahren des CT-Tisches und das Verschieben der Plattform jeweils mit einer vom Betrag her gleichen Geschwindigkeit durchgeführt werden, sodass der CT-Tisch relativ zu der Standfläche stationär bleibt.

Vorzugsweise werden also das Einfahren des CT-Tisches und das Verschieben der Plattform in zueinander entgegengesetzte Richtung mit jeweils einer vom Betrag her gleichen Geschwindigkeit durchgeführt, sodass der CT-Tisch relativ zu einer Standfläche eines Großtieres stationär bleibt.

Bevorzugt umfasst das Verfahren weiterhin ein
- Anheben oder Absenken der Plattform relativ zu einer Standfläche eines Großtieres.

Die Erfindung soll nun anhand eines Ausführungsbeispiels in Bezug auf die Figuren näher erläutert werden. Von den Figuren zeigt:
- Fig. 1:: eine Computertomographievorrichtung zum Untersuchen eines Körperteils eines Großtieres;
- Fig. 2:: eine Computertomographie-Untersuchungsanordnung aufweisend eine in einer Bodengrube angeordnete Computertomographievorrichtung in einer Seitenansicht;
- Fig. 3:: eine Computertomographievorrichtung mit Haltevorrichtung und Ablage;
- Fig. 4:: eine Computertomographievorrichtung zum Untersuchen eines Körperteils eines Großtieres.

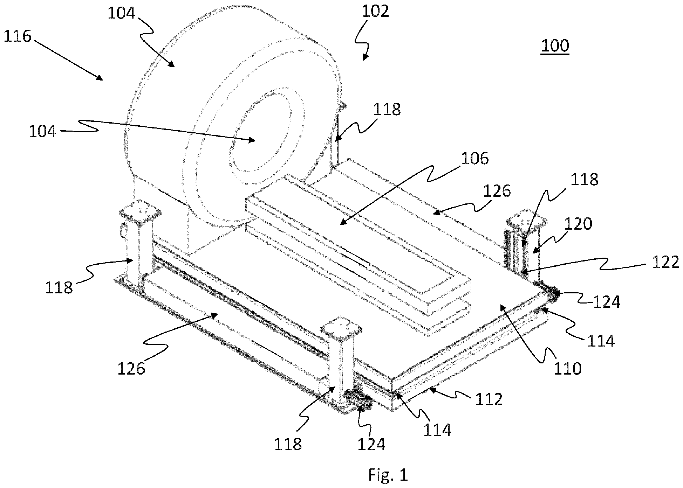

In **Figur 1** ist eine Computertomographievorrichtung 100 zum Untersuchen eines Körperteils eines Großtieres gezeigt.

Die Computertomographievorrichtung 100 weist ein CT-Gerät 102 auf, welches eine ringförmige Gantry 104 und einen CT-Tisch 106 umfasst. Die Gantry 104 ist als Ringtunnel ausgebildet, in dem eine CT-Röhre und gegenüber von der CT-Röhre ein Detektor (beide nicht gezeigt) angeordnet sind. Mittig weist die ringförmige Gantry 104 eine Öffnung 108 auf. Der CT-Tisch 106 und die Gantry 104 sind zueinander beweglich miteinander verbunden, sodass der CT-Tisch 106 in die Öffnung 108 der ringförmigen Gantry 104 eingefahren werden kann. Ein wie in Figur 1 gezeigtes CT-Gerät 102 ist grundsätzlich im freien Handel verfügbar.

Die Computertomographievorrichtung 100 weist weiterhin eine Plattform 110 auf, auf der das CT-Gerät 102 montiert ist. Die Plattform 110 ist auf einer Bodenplatte 112 angeordnet, wobei sich zwischen der Bodenplatte 112 und der Plattform110 ein Schienensystem 114 befindet. Das Schienensystem 114 ermöglicht, dass die Bodenplatte 112 und die Plattform 110 zueinander vergleichsweise reibungsarm in diejenigen Richtungen hin und her verschoben werden kann, in die auch der CT-Tisch 106 beweglich ist. Das Schienensystem 114 ist ein Element einer Verschiebevorrichtung, welche weiterhin einen Verschiebemotor (nicht gezeigt) und eine von dem Verschiebemotor antreibbare Gewindespindel (nicht gezeigt) aufweist, wobei auf der Gewindespindel ein Gegengewinde (nicht gezeigt) verschiebbar gelagert ist. Das Gegengewinde ist fest mit der Plattform 110 verbunden, sodass im Betrieb des Verschiebemotors eine Rotation der Gewindespindel in eine translatorische Verschiebung der Plattform 110 relativ zu einer Standfläche eines Großtieres umgewandelt wird. Die Standfläche befindet sich hierbei auf derjenigen Seite 116 der Gantry 104, die dem CT-Tisch 106 gegenüberliegt. Die Standfläche, auf der ein Großtier während einer CT-Untersuchung stationär steht, befindet sich also außerhalb der Computertomographievorrichtung 100. Durch die Verschiebevorrichtung kann die Plattform 110 mit dem CT-Gerät 104 also relativ zu einer Standfläche eines Großtieres verschoben werden.

Die Computertomographievorrichtung 100 weist wenigstens einen Betriebszustand auf, in dem die Plattform 110 derart verschoben wird, dass der CT-Tisch 106 während einer Relativbewegung zur Gantry relativ zu der Standfläche stationär bleibt. Beispielsweise können in diesem Betriebszustand insbesondere der CT-Tisch 106 und die Plattform 104 derart bewegen, dass die Bewegung des CT-Tisches 106 durch eine Bewegung der Plattform 110 in die dazu entgegengesetzte Richtung, für einen außenstehenden Beobachter und insbesondere für ein auf der Standfläche befindliches Großtier kompensiert wird. Für einen außenstehenden Betrachter ergibt sich dann ein Bild als wenn die Gantry über den CT-Tisch hinwegfahren würde während der CT-Tisch stillsteht.

In diesem Betriebszustand würde dann der CT-Tisch 106 wie vorgesehen in die Öffnung 108 der Gantry104 einfahren und damit eigentlich auf ein dahinterstehendes Großtier zu. Die Plattform 110 mit dem CT-Gerät 102 bewegt sich jedoch mit einer vom Betrag her gleichen Geschwindigkeit von der Standfläche des Großtieres weg. Dadurch bleibt ein Körperteil, welches auf den CT-Tisch gestellt oder gelegt ist, relativ zu der Standfläche in unbewegter Position, während die Gantry 104 über den Körperteil hinwegzufahren scheint. Für einen außenstehenden Beobachter scheint der der CT-Tisch 106 also in diesem Betriebszustand stillzustehen und die Gantry 104 scheint über den CT-Tisch 106, auf dem ein zu untersuchendes Körperteil eines Großtieres gelegt oder gestellt sein kann, hinwegzufahren.

In der gezeigten Ausführungsform weist das CT-Gerät 102 einen CT-Motor (nicht gezeigt) auf, der ausgebildet den CT-Tisch 106 motorbetrieben zu verfahren. In der gezeigten Ausführungsform sind der CT-Motor und der Verschiebemotor derart gekoppelt, dass ein Startsignal am CT-Motor abgegriffen und an den Verschiebemotor übertragen wird, sobald der CT-Tisch durch CT-Motor angetrieben in Richtung der Öffnung 108 der Gantry 104 fährt. In den Verschiebemotor oder in ein Steuergerät des Verschiebemotors sind eine Anzahl vordefinierter Geschwindigkeiten einprogrammiert und eine der einprogrammierten Geschwindigkeiten für eine CT-Untersuchung ausgewählt. Entsprechend wird für eine CT-Untersuchung ein CT-Protokoll so ausgewählt, dass sich der CT-Tisch während einer CT-Untersuchung mit der ausgewählten Geschwindigkeit des Verschiebemotors bewegt. Der CT-Motor und der Verschiebemotor sind also an sich unabhängig voneinander implementiert. Jedoch werden die Geschwindigkeiten der beiden Motoren über das Startsignal synchronisiert. Die Plattform 110 wird dann durch den Verschiebemotor aufgrund des empfangenen Startsignals mit vom Betrag her gleicher Geschwindigkeit im Wesentlichen zeitgleich in eine Richtung verschoben, die der Richtung in die der CT-Tisch 106 in die Öffnung 108 der ringförmigen Gantry 104 einfährt entgegengesetzt ist. Für einen außenstehenden Beobachter scheint der CT-Tisch106 dann stillzustehen und die Gantry 104 scheint über den CT-Tisch 106 und insbesondere über ein zu untersuchendes Körperteil eines Großtieres hinwegzufahren.

Alternativ zu einer Kopplung durch Abgreifen eines Startsignals, kann die Kopplung des CT-Motors und des Verschiebemotors derart gestaltet sein, dass der CT-Antrieb und der Verschiebeantrieb mechanisch miteinander verbunden sind. In einer nicht gezeigten Ausführungsform sind der CT-Antrieb und der Verschiebeantrieb über eine Spindel miteinander verbunden. Die Bewegung wird dann direkt an dem CT-Tisch abgegriffen und mechanisch über eine Spindel an den Verschiebeantrieb übermittelt werden. Dadurch wird die Bewegung der Plattform direkt an die Bewegung CT-Tisches gekoppelt. Vorteilhafterweise kann so eine Synchronisation der Bewegungen des CT-Tisches und der Plattform weiter verbessert werden. Wird der CT-Tisch beispielsweise händisch oder über die Steuerkonsole bewegt, so bewegt sich die Plattform mit vom Betrag her gleicher Geschwindigkeit und insbesondere in eine Richtung, die der Einfahrrichtung des CT-Tisches in die Gantry entgegengesetzt ist. Dadurch scheint die Gantry für einen außenstehenden Beobachter über den stillstehenden CT-Tisch hinwegzufahren.

In einer weiteren hier nicht gezeigten Ausführungsform sind der CT-Antrieb und der Verschiebeantrieb derart gekoppelt, dass die Geschwindigkeit und/oder die Position des CT-Tisches und/oder der Plattform durch einen entsprechenden Geschwindigkeits- oder Positionssensor gemessen werden. Ein Geschwindigkeitssignal und/oder Positionssignal, welche entsprechend die gemessene Geschwindigkeit oder Position repräsentieren, kann dann an den CT-Motor und/oder den Verschiebemotor übertragen und verwendet werden, den CT-Motor und/oder den Verschiebemotor derart zu regeln, dass sich CT-Tisch und Plattform mit vom Betrag her gleicher Geschwindigkeit in zueinander entgegengesetzte Richtungen bewegen.

Seitlich an der Bodenplatte 112 sind vier Säulen 118 angeordnet in denen jeweils eine Gewindespindel 120 angeordnet ist. Die Gewindespindeln 120 sind Elemente einer Hebevorrichtung und in Bezug auf die Bodenplatte 112 senkrecht in Richtung der Plattform 110 seitlich an der Bodenplatte 112 angeordnet. Jede der Gewindespindeln 120 ist über jeweils ein verschiebbar gelagertes Gegengewinde 122 mit der Bodenplatte 112 verbunden. Die Gegengewinde 122 sind also fest mit der Bodenplatte 112 verbunden und jeweils verschiebbar auf einer Gewindespindel 120 gelagert. Wenn die Gewindespindeln 120 der Hebevorrichtung angetrieben werden, kann so die Bodenplatte 112 und die auf der Bodenplatte 112 angeordnete Plattform 110 mit dem CT-Gerät 102 angehobenen oder abgesenkt werden. Zum Antreiben der Gewindespindeln 120 der Hebevorrichtung sind zwei der Gewindespindeln 120 jeweils mit einem Hebemotor 124 verbunden.

Die Gegengewinde 122 der Hebevorrichtung, die in Säulen 118 auf derselben Längsseite der Bodenplatte angeordnet sind, sind durch eine Synchronisationsvorrichtung 126 mechanisch miteinander verbunden. Die Synchronisationsvorrichtungen 126 sind als durchgehende Verbindungselemente ausgebildet, und koppeln jeweils zwei Gegengewinde einer Längsseite der Bodenplatte mechanisch miteinander. Die Verbindungselemente 126 sind auch mit der Bodenplatte 112 verbunden, sodass die Verbindung der Gegengewinde 122 mit der Bodenplatte 112 zusätzlich unterstützt wird.

Wenn die Gewindespindeln 120 der Hebevorrichtung, die direkt von dem Hebemotor angetrieben werden können, im Motorbetrieb angetrieben werden und sich die darauf verschiebbar gelagerten Gegenwinde 122 jeweils entlang der Gewindespindeln 120 verschieben, verschieben sich die über die Synchronisationsvorrichtungen 126 mit den Gegengewinden 118 verbundenen weiteren Gegengewinde 118 synchron mit den Gegengewinden 118 der angetriebenen Gewindespindeln 120. Dadurch kann die Bodenplatte 112 gleichmäßig angehoben oder abgesenkt werden.

In hier nicht gezeigten Ausführungsformen weist die Hebevorrichtung zusätzlich oder alternativ zu einem Spindelantrieb wenigstens einen hydraulischen Druckzylinder auf. In einer solchen Ausführungsform ist ein Druckzylinder derart in Bezug auf eine Bodenplatte angeordnet ist, dass die Bodenplatte durch Druckbeaufschlagung des Druckzylinders relativ zu einer Standfläche angehoben oder abgesenkt werden kann.

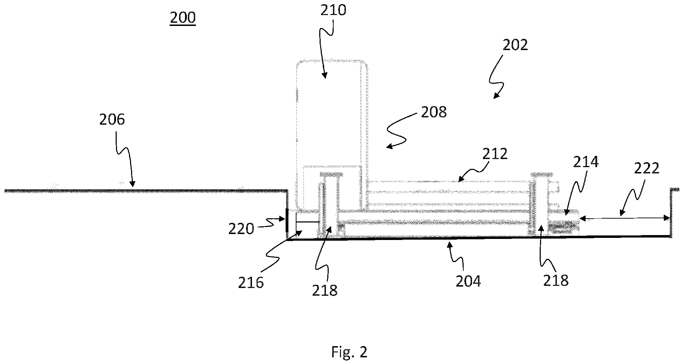

In **Figur 2** ist eine Computertomographie-Untersuchungsanordnung 200 aufweisend eine in einer Bodengrube angeordnete Computertomographievorrichtung 202, eine Bodengrube 204 und eine Standfläche für ein Großtier 206 in einer Seitenansicht gezeigt.

Die Computertomographievorrichtung 202 ist analog zu der in Bezug auf Figur 1 beschriebenen Computertomographievorrichtung ausgebildet und umfasst insbesondere ein CT-Gerät 208 mit einer Gantry 210 und einem CT-Tisch 212, der mit einem CT-Motor in eine Öffnung der ringförmigen Gantry 208 eingefahren werden kann. Das CT-Gerät 208 ist auf einer Plattform 214 montiert, die auf einer Bodenplatte 216 angeordnet ist. Die Plattform mit dem CT-Gerät 208 kann durch eine Verschiebevorrichtung (nicht gezeigt) relativ zu der Standfläche 206 in diejenige Richtung hin und her verschoben werden, in die auch der CT-Tisch 212 beweglich ist. Die Bodenplatte 216 kann durch eine Hebevorrichtung 218 angehoben und abgesenkt werden, sodass das auf der Plattform 214 montierte CT-Gerät 208 mittels der Hebevorrichtung 218 und der Verschiebevorrichtung relativ zu der Standfläche 206 sowohl vertikal angehoben und abgesenkt als auch horizontal verschoben werden kann.

Wie aus Figur 2 hervorgeht, befindet sich die Standfläche 206 außerhalb der Bodengrube 204 und zwar angrenzend an diejenige Seitenwand 220 der Bodengrube 206, die der Gantry 210 der Computertomographievorrichtung 202 am nächsten ist. Die Bodengrube 204 weist eine Tiefe auf die so bemessen ist, dass sich der CT-Tisch 212 des CT-Geräts208, wenn das CT-Gerät 202 wie in Figur 2 dargestellt durch die Hebevorrichtung 218 vollständig abgesenkt ist, auf derselben Höhe wie die Standfläche 206 befindet.

In dem gezeigten Ausführungsbeispiel kann der Kopf eines Großtieres oberhalb der Gantry positioniert werden, sodass sich dann der Rumpf des Großtieres unmittelbar vor der Gantry befindet. In diesem vollständig abgesenkten Zustand kann dann beispielsweise ein Vorderbein des Großtieres schräg nach vorne auf den CT-Tisch 212 gestellt werden, sodass die Gantry 210 während einer CT-Untersuchung relativ zu der Standfläche 206 des Großtieres über das Vorderbein hinwegzufahren scheint. Ein Hinterbein eines Großtieres kann entsprechend von dem Großtier aus schräg nach hinten auf den CT-Tisch 212 gestellt werden.

Die Bodengrube 204 weist eine laterale Ausdehnung auf, die es erlaubt, dass die Plattform 214 relativ zu der Bodenplatte 216 durch eine Verschiebevorrichtung verschoben werden kann, ohne an die Seitenwände der Bodengrube 204 zu stoßen. Die Bodengrube 204 weist also insbesondere an den entsprechenden Seiten der Computertomographievorrichtung 202 einen vergleichsweise größeren Abstand 222 zu der Computertomographievorrichtung 202 auf. An den beiden Längsseiten der Computertomographievorrichtung 202, entlang der während einer CT-Untersuchung keine Verschiebung der Plattform214 relativ zu der Bodenplatte 216 stattfindet, können die Seitenwände der Bodengrube 204 vergleichsweise nah an die Computertomographievorrichtung 202 angrenzen.

Durch die Hebevorrichtung 218 kann das CT-Gerät 210 bis zu zwei Meter angehoben werden. In einer hier nicht gezeigten Ausführungsform kann ein CT-Gerät über zwei Meter hinaus, beispielsweise bis zu drei Meter, angehoben werden. Im angehobenen Zustand kann dann beispielsweise eine CT-Untersuchung des Kopfes und des Halses eines Großtieres durchgeführt werden. Mit der gezeigten Computertomographie-Untersuchungsanordnung 200 können also sowohl die Gliedmaßen als auch der Kopf oder der Hals eines stationär stehenden Großtieres untersucht werden. Insbesondere können mit der gezeigten Computertomographie-Untersuchungsanordnung 200 mit demselben handelsüblichen CT-Gerät 202 nacheinander Kopf, Hals und Vorderbeine eines stationär stehenden Großtieres untersucht werden, ohne dass das Großtier seine stationäre Position auf der Standfläche 206 verändern oder aufgeben muss.

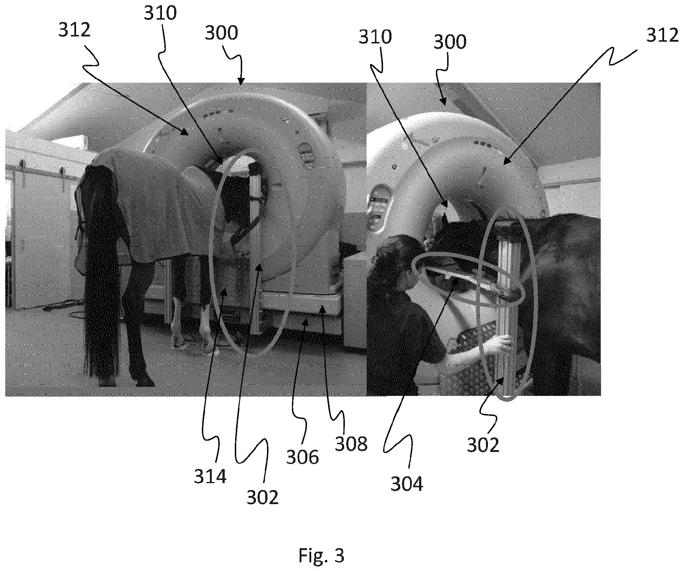

**Figur 3** zeigt zwei Ansichten einer Computertomographievorrichtung 300 mit einem Untersuchungsstand, der eine Haltevorrichtung 302 und eine Ablage 304 umfasst.

Die Ablage 304 ist an der Haltevorrichtung 302 angebracht und derart gestaltet, dass ein Körperteil eines Großtieres, hier eines Pferdes, auf der Ablage 304 abgelegt werden kann. Die Haltevorrichtung ist an einer Bodenplatte 306 angebracht, sodass sich Haltevorrichtung 302 und Ablage 304 während eines Verschiebens der Plattform 308 durch eine Verschiebevorrichtung nicht mitbewegen. Wenn allerdings die Bodenplatte 306 durch eine Hebevorrichtung angehoben oder abgesenkt wird, bewegen sich die Haltevorrichtung 302 und die Ablage 304 gemeinsam mit der Bodenplatte 306, sodass die Ablage 304 auf eine bestimmte Höhe in Bezug auf das zu untersuchende Großtier eingestellt werden kann. Die Ablage 304 kann so an die Höhe eines zu untersuchenden Großtieres und insbesondere eines zu untersuchenden Körperteils, beispielsweise an die Höhe eines Pferdekopfes oder Halses, angepasst werden.

Die Ablage 304 ist jedoch in einer Höhe in Bezug auf das CT-Gerät angeordnet, dass die Ablage 304 durch ein Verschieben der Plattform 308 mit dem CT-Gerät in die Öffnung 310 der ringförmigen Gantry 312 eingefahren werden kann, insbesondere soweit, dass die Ablage 304 durch die Öffnung 310 der ringförmigen Gantry 312 hindurch ragt. Während einer CT-Untersuchung scheint die Gantry 312 also für einen außenstehenden Betrachter über die Ablage 304 hinwegzufahren.

An der Computertomographievorrichtung 300 ist auf derjenigen Seite, die der Standfläche eines Großtieres zugewandt ist eine Metall-Konstruktion 314 angebracht. Die Metall-Konstruktion 314 dient als Schutz für das CT-Gerät vor einem Einwirken des Großtieres, beispielsweise einem Tritt, auf das CT-Gerät.

In der gezeigten Ausführungsform einer Computertomographievorrichtung 300 mit einem Untersuchungsstand kann die Ablage auf der dem CT-Tisch zugewandten Seite mit dem CT-Tisch mechanisch verbunden werden. Wenn der CT-Tisch ausgekoppelt und dadurch in Bezug auf das übrige CT-Gerät frei beweglich ist, wird der CT-Tisch nicht mit der Plattform und dem übrigen CT-Gerät verschoben, wenn die Plattform durch die Verschiebevorrichtung verschoben wird. Der CT-Tisch wird dann durch die mechanische Verbindung mit der Ablage für einen außenstehenden Beobachter unbeweglich gehalten. Wenn das CT-Gerät zusammen mit der Plattform durch die Verschiebevorrichtung verschoben wird, scheint der CT-Tisch durch die mechanische Verbindung mit der Ablage relativ zu der Standfläche stationär zu bleiben, während die Gantry über den CT-Tisch relativ zu der Standfläche hinwegzufahren scheint.

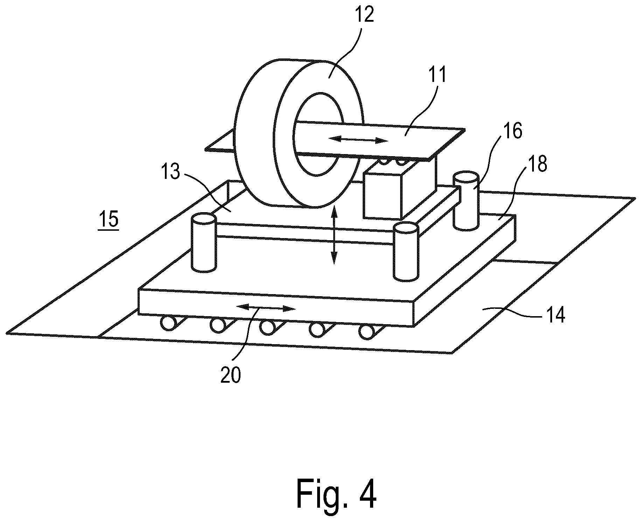

In **Figur 4** ist eine Computertomographievorrichtung 15 zum Untersuchen eines Körperteils eines Großtieres gezeigt. Die Computertomographievorrichtung 15 umfasst ein CT-Gerät, welches eine Gantry 12 und einen CT-Tisch 11 umfasst. Das CT-Gerät ist auf einer Bodenplatte 13 angeordnet, die mittels einer Hebevorrichtung 16 vertikal angehoben oder abgesenkt werden kann. Die Gantry 12 ist auf der Bodenplatte 13 unbeweglich angeordnet. Der CT-Tisch 16 ist auf der Bodenplatte 13 horizontal beweglich angeordnet und kann sich relativ zu der Gantry und/oder der Bodenplatte horizontal bewegen. Die Bodenplatte 13 und die Hebevorrichtung 16 sind auf einer Plattform 18 angeordnet. Die Plattform 18 ist auf festem Untergrund 14 beispielsweise in einer Bodengrube angeordnet und kann mittels einer Verschiebevorrichtung 20 horizontal in Bezug auf den Untergrund 14 verschoben werden.

### Bezugszeichenliste

- 11: CT-Tisch
- 12: Gantry
- 13: Bodenplatte
- 14: Untergrund
- 15: Computertomographievorrichtung
- 16: Hebevorrichtung
- 18: Plattform
- 20: Verschiebevorrichtung
- 100: Computertomographievorrichtung
- 102: CT-Gerät
- 104: Gantry
- 106: CT-Tisch
- 108: Öffnung
- 110: Plattform
- 112: Bodenplatte
- 114: Schienensystem
- 116: Seite der Gantry, die dem CT-Tisch gegenüberliegt
- 118: Säulen der Hebevorrichtung
- 120: Gewindespindeln der Hebevorrichtung
- 122: Gegengewinde der Hebevorrichtung
- 124: Hebemotor
- 126: Synchronisationsvorrichtungen
- 200: Computertomographie-Untersuchungsanordnung
- 202: Computertomographievorrichtung
- 204: Bodengrube
- 206: Standfläche für ein Großtier
- 208: CT-Gerät
- 210: Gantry
- 212: CT-Tisch
- 214: Plattform
- 216: Bodenplatte
- 218: Hebevorrichtung
- 220: Seitenwand
- 222: Abstand zwischen Bodenplatte und Seitenwand
- 300: Computertomographievorrichtung
- 302: Haltevorrichtung
- 304: Ablage
- 306: Bodenplatte
- 308: Plattform
- 310: Öffnung
- 312: Gantry
- 314: Metall-Konstruktion

## Patentansprüche

1. Computertomographievorrichtung (100) zum Untersuchen eines Körperteils eines Großtieres aufweisend:
- ein Computertomographie (CT)-Gerät (102) mit einer ringförmigen Gantry (104) und einen CT-Tisch (106) zur Aufnahme eines zu untersuchenden Körperteils eines Großtieres,
- und eine Plattform (110), auf der das CT-Gerät (102) montiert ist,
wobei die Gantry (104) relativ zu der Plattform unbeweglich ist, und
wobei der CT-Tisch (106) relativ zu der Plattform und zu der Gantry horizontal beweglich mit der Gantry und/oder der Plattform derart verbunden ist, dass der CT-Tisch (106) in eine Öffnung (108) der ringförmigen Gantry (104) eingefahren werden kann,
**dadurch gekennzeichnet, dass**
die Computertomographievorrichtung (100) eine Verschiebevorrichtung umfasst, die mit der Plattform (110) verbunden und ausgebildet ist, die Plattform (110) mit dem montierten CT-Gerät (102) relativ zu einer außerhalb der Plattform (110) befindlichen Standfläche eines Großtieres horizontal zu verschieben,
wobei die die Computertomographievorrichtung (100) einen Untersuchungsstand aufweist, der eine Haltevorrichtung (302) und eine von der Haltevorrichtung (302) gehaltene Ablage (304) umfasst, wobei der Untersuchungsstand derart angeordnet ist, dass die Ablage (304) während eines Verschiebens der Plattform durch die Verschiebevorrichtung nicht mitbewegt wird, und
wobei die Ablage (304) auf der dem CT-Tisch zugewandten Seite mit dem CT-Tisch mechanisch verbunden ist, und wobei der CT-Tisch ausgekoppelt und dadurch in Bezug auf das übrige CT-Gerät frei beweglich ist, sodass der mit der Ablage (304) verbundene CT-Tisch, wenn das auf der Plattform montierte CT-Gerät zusammen mit der Plattform durch die Verschiebevorrichtung verschoben wird, durch die mechanische Verbindung mit der Ablage relativ zu der Standfläche während einer Relativbewegung zur Gantry stationär bleibt und die Haltevorrichtung (302) und die Ablage (304) beim Verschieben der Plattform nicht mitbewegt werden.

2. Computertomographievorrichtung (100) nach Anspruch 1, die zusätzlich eine Hebevorrichtung aufweist, wobei die Hebevorrichtung ausgebildet ist, die Plattform (110) relativ zu der Standfläche eines Großtieres vertikal anzuheben oder abzusenken, sodass das auf der Plattform (110) montierte CT-Gerät (102) mittels der Hebevorrichtung und der Verschiebevorrichtung relativ zu der Standfläche sowohl vertikal angehoben und abgesenkt als auch horizontal verschoben werden kann.

3. Computertomographievorrichtung (100) nach Anspruch 1 oder 2, wobei die Verschiebevorrichtung ein Schienensystem (114) umfasst, welches auf einer Bodenplatte (112) angeordnet ist, und wobei die Plattform (110) mit dem montierten CT-Gerät (102) auf dem Schienensystem (114) angeordnet ist, sodass die Bodenplatte (112) und die Plattform (110) durch das Schienensystem (114) relativ zueinander beweglich miteinander verbunden sind.

4. Computertomographievorrichtung (100) nach wenigstens einem der Ansprüche 1 bis 3, wobei die Verschiebevorrichtung einen Verschiebemotor umfasst, der ausgebildet ist, einen Verschiebeantrieb zum Verschieben der Plattform (110) relativ zu der Standfläche eines Großtieres bereitzustellen, und wobei die Verschiebevorrichtung eine Gewindespindel und ein auf der Gewindespindel verschiebbar gelagertes Gegengewinde umfasst, wobei der Verschiebemotor zum Antreiben der Gewindespindel mit der Gewindespindel verbunden ist, und das Gegengewinde an der Plattform (110) angeordnet ist, sodass im Betrieb des Verschiebemotors eine Rotation der Gewindespindel in eine translatorische Verschiebung der Plattform (110) relativ zu der Standfläche umgewandelt wird.

5. Computertomographievorrichtung (100) nach wenigstens einem der Ansprüche 1 bis 4, wobei das CT-Gerät einen CT-Antrieb zum motorbetriebenen Einfahren des CT-Tisches (106) in die Gantry (104) umfasst, und wobei ein Verschiebeantrieb und der CT-Antrieb durch eine Kopplung mechanisch miteinander gekoppelt sind, wobei die Kopplung derart gestaltet ist, dass der CT-Antrieb direkt abgegriffen und an den Verschiebeantrieb übertragen wird.

6. Computertomographievorrichtung (100) nach Anspruch 5, wobei die mechanische Kopplung des CT-Antriebs und des Verschiebeantriebs derart gestaltet ist, dass sich der CT-Tisch und die Plattform (110) im Betrieb mit vom Betrag her gleicher Geschwindigkeit in jeweils zueinander entgegengesetzte Richtungen verschieben.

7. Computertomographievorrichtung (100) nach wenigstens einem der Ansprüche 2 bis 6, wobei die Plattform (110) mit dem CT-Gerät (102) auf einer Bodenplatte (112) angeordnet ist und die Hebevorrichtung eine Mehrzahl Gewindespindeln (120) umfasst, die in Bezug auf die Bodenplatte (112) senkrecht in Richtung der Plattform (110) seitlich an der Bodenplatte (112) angeordnet und jeweils über ein verschiebbar gelagertes Gegengewinde (122) mit der Bodenplatte (112) verbunden sind.

8. Computertomographievorrichtung nach Anspruch 7, wobei die Hebevorrichtung wenigstens einen Hebemotor (124) umfasst, der zum Antreiben wenigstens einer der Mehrzahl der Gewindespindeln (120) der Hebevorrichtung mit einer der Gewindespindeln (120) verbunden und ausgebildet ist, einen Hebeantrieb zum Anheben oder Absenken der Plattform (110) relativ zu der Standfläche eines Großtieres bereitzustellen.

9. Computertomographievorrichtung nach wenigstens einem der Ansprüche 2 bis 8, wobei die Plattform mit dem CT-Gerät auf einer Bodenplatte angeordnet ist und die Hebevorrichtung wenigstens einen hydraulischen Druckzylinder umfasst, der derart in Bezug auf die Bodenplatte angeordnet ist, dass die Bodenplatte durch Druckbeaufschlagung des Druckzylinders relativ zu der Standfläche angehoben oder abgesenkt werden kann.

10. Computertomographievorrichtung (300) nach wenigstens einem der Ansprüche 1 bis 9, bei der das CT-Gerät ein handelsübliches CT-Gerät ist, bei dem die Gantry in Bezug auf den CT-Tisch um 180° gedreht ist.

11. Computertomographievorrichtung (300) nach Anspruch 1, die eine Bodenplatte umfasst, auf der die Plattform mit dem montierten CT-Gerät angeordnet ist, wobei die Haltevorrichtung (302) an der Bodenplatte angebracht ist, sodass sich die Haltevorrichtung (302) und die Ablage (304) gemeinsam mit der Bodenplatte bewegen, wenn diese durch eine Hebevorrichtung relativ zu der Standfläche angehoben oder abgesenkt wird.

12. Computertomographie-Untersuchungsanordnung (200) aufweisend eine Computertomographievorrichtung (202) nach wenigstens einem der Ansprüche 1 bis 11, eine Standfläche (204) für ein Großtier und eine Bodengrube (206), wobei die Computertomographievorrichtung (202) in der Bodengrube (204) angeordnet ist, sodass sich wenigstens ein Teil der Computertomographievorrichtung (202) auf einem im Vergleich zu der Standfläche (204) niedrigeren Niveau befindet, und wobei sich die Standfläche (204) außerhalb der Bodengrube (206) an diejenige Seitenwand (220) der Bodengrube (206) angrenzend befindet, die der Gantry (210) der Computertomographievorrichtung (202) am nächsten ist.

13. Verfahren zum Untersuchen eines Körperteils eines Großtieres mit einer Computertomographievorrichtung, wobei die Computertomographievorrichtung aufweist:
- ein Computertomographie (CT)-Gerät mit einer ringförmigen Gantry und einem CT-Tisch zur Aufnahme eines zu untersuchenden Körperteils eines Großtieres, und
- eine Plattform, auf der das CT-Gerät montiert ist,
wobei die Gantry (104) relativ zu der Plattform unbeweglich ist,
wobei der CT-Tisch (106) relativ zu der Plattform und zu der Gantry horizontal beweglich mit der Gantry und/oder der Plattform derart verbunden ist,
dass der CT-Tisch (106) in eine Öffnung (108) der ringförmigen Gantry (104) eingefahren werden kann,
wobei das Verfahren die Schritte aufweist:
- Positionieren eines Großtieres auf einer außerhalb der Plattform befindlichen Standfläche,
- Auflegen eines zu untersuchenden Körperteils auf den CT-Tisch oder eine Ablage des CT-Geräts,
- Verschieben der Plattform mit dem CT-Gerät relativ zu der Standfläche,
wobei die Verschiebevorrichtung ausgebildet ist, in wenigstens einem Betriebszustand die Plattform (110) mit dem CT-Gerät (102) zu verschieben und
der CT-Tisch ausgekoppelt und dadurch in Bezug auf das übrige CT-Gerät frei beweglich ist, sodass der mit der Ablage (304) verbundene CT-Tisch, wenn das auf der Plattform montierte CT-Gerät zusammen mit der Plattform durch die Verschiebevorrichtung verschoben wird, durch die mechanische Verbindung mit der Ablage relativ zu der Standfläche während einer Relativbewegung zur Gantry stationär bleibt und die Haltevorrichtung (302) und die Ablage (304) beim Verschieben der Plattform nicht mitbewegt werden.

14. Verfahren nach Anspruch 13, wobei in dem Betriebszustand der CT-Tisch relativ zu der Plattform in die Öffnung der ringförmigen Gantry in eine erste Richtung einfährt, und die Plattform in eine zweite Richtung, die der ersten Richtung entgegengesetzt ist relativ zu der Standfläche verschoben wird.

## Claims

1. A computer tomography apparatus (100) for examining a body part of a large animal comprising:
- a computerised tomography (CT) device (102) with a ring-shaped gantry (104) and a CT table (106) for recording a part of the body of a large animal to be examined,
- and a platform (110) on which the CT device (102) is mounted,
the gantry (104) being immovable relative to the platform, and
wherein the CT table (106) is horizontally movably connected to the gantry and/or the platform relative to the platform and to the gantry such that the CT table (106) can be retracted into an opening (108) of the annular gantry (104),
**characterised in that**
the computer tomography apparatus (100) comprises a displacement apparatus connected to the platform (110) and adapted to horizontally displace the platform (110) with the mounted CT device (102) relative to a standing surface for a large animal located outside the platform (110),
wherein the computed tomography apparatus (100) has an examination stand comprising a support device (302) and a shelf (304) supported by the support device (302), the examination stand being arranged such that the shelf (304) is not moved during displacement of the platform by the displacement apparatus, and
wherein the shelf (304) is mechanically connected to the CT table on the side facing the CT table, and wherein the CT table is disengaged and thereby freely movable with respect to the remainder of the CT device, such that the CT table connected to the shelf (304), when the CT device mounted on the platform is displaced together with the platform by the displacement apparatus, remains stationary relative to the standing surface during relative movement to the gantry due to the mechanical connection to the shelf, and the support device (302) and the shelf (304) are not moved when the platform is displaced.

2. The computer tomography apparatus (100) of claim 1, further comprising a lifting apparatus, wherein the lifting apparatus is adapted to vertically raise or lower the platform (110) relative to the standing surface for a large animal so that the CT device (102) mounted on the platform (110) can be both vertically raised and lowered and horizontally displaced relative to the standing surface by means of the lifting apparatus and the displacement device.

3. Computed tomography apparatus (100) according to claim 1 or 2, wherein the displacement device comprises a rail system (114) arranged on a base plate (112), and wherein the platform (110) with the mounted CT device (102) is arranged on the rail system (114) so that the base plate (112) and the platform (110) are movably connected to each other relative to each other by the rail system (114).

4. Computed tomography apparatus (100) according to at least one of claims 1 to 3, wherein the displacement apparatus comprises a displacement motor configured to provide a displacement drive for displacing the platform (110) relative to the standing surface for a large animal, and wherein the displacement apparatus comprises a threaded spindle and a counter thread displaceably mounted on the threaded spindle, wherein the displacement motor is connected to the threaded spindle for driving the threaded spindle, and the mating thread is arranged on the platform (110) such that, in operation of the displacement motor, rotation of the threaded spindle is converted into translational displacement of the platform (110) relative to the standing surface.

5. Computed tomography apparatus (100) according to at least one of claims 1 to 4, wherein the CT device comprises a CT drive for motor-driven retraction of the CT table (106) into the gantry (104), and wherein a translation drive and the CT drive are mechanically coupled to each other by a coupling, the coupling being such that the CT drive is directly tapped and transmitted to the translation drive.

6. The computed tomography apparatus (100) of claim 5, wherein the mechanical coupling of the CT drive and the translation drive is such that, in operation, the CT table and the platform (110) translate in opposite directions at the same rate.

7. Computer tomography apparatus (100) according to at least one of claims 2 to 6, wherein the platform (110) with the CT device (102) is arranged on a base plate (112) and the lifting apparatus comprises a plurality of threaded spindles (120) which are arranged laterally on the base plate (112) perpendicularly with respect to the base plate (112) in the direction of the platform (110) and are each connected to the base plate (112) via a displaceably mounted counter-thread (122).

8. The computed tomography apparatus of claim 7, wherein the lifting apparatus comprises at least one lifting motor (124) connected to one of the threaded spindles (120) of the lifting apparatus for driving at least one of the plurality of threaded spindles (120) and adapted to provide a lifting drive for raising or lowering the platform (110) relative to the standing surface for a large animal.

9. computed tomography apparatus according to at least one of claims 2 to 8, wherein the platform with the CT device is arranged on a base plate and the lifting apparatus comprises at least one hydraulic pressure cylinder arranged with respect to the base plate in such a way that the base plate can be raised or lowered relative to the base plate by applying pressure to the pressure cylinder.

10. Computed tomography apparatus (300) according to at least one of claims 1 to 9, wherein the CT device is a commercially available CT device in which the gantry is rotated 180° with respect to the CT table.

11. The computed tomography apparatus (300) of claim 1, comprising a base plate on which the platform with the mounted CT device is arranged, wherein the support device (302) is attached to the base plate so that the support device (302) and the shelf (304) move together with the base plate when the latter is raised or lowered relative to the base plate by a lifting apparatus.

12. A computed tomography examination arrangement (200) comprising a computed tomography apparatus (202) according to at least one of claims 1 to 11, a standing surface (204) for a large animal and a floor pit (206), wherein the computed tomography apparatus (202) is located in the floor pit (204), such that at least a portion of the computed tomography apparatus (202) is at a lower level relative to the standing surface (204), and wherein the standing surface (204) is located outside the floor pit (206) adjacent that side wall (220) of the floor pit (206) which is closest to the gantry (210) of the computed tomography apparatus (202).

13. A method of examining a body part of a large animal with a computed tomography apparatus, the computed tomography apparatus comprising:
- a computed tomography (CT) device with a ring-shaped gantry and a CT table for imaging a part of the body of a large animal to be examined, and
- a platform on which the CT device is mounted,
wherein the gantry (104) is immobile relative to the platform,
wherein the CT table (106) is horizontally movable relative to the platform and to the gantry and is connected to the gantry and/or the platform in such a manner,
that the CT table (106) can be retracted into an opening (108) of the annular gantry (104),
the method comprising the steps of:
- Positioning a large animal on a standing surface outside the platform,
- Placing a body part to be examined on the CT table or a shaft of the CT unit,
- Shifting the platform with the CT device relative to the standing surface,
wherein the displacement apparatus is configured to displace the platform (110) with the CT device (102) in at least one operating state, and
the CT table is disengaged and thereby freely movable with respect to the rest of the CT device, so that when the CT device mounted on the platform is displaced together with the platform by the displacement apparatus, the CT table connected to the shelf (304) remains stationary relative to the standing surface during relative movement to the gantry due to the mechanical connection to the shelf, and the support device (302) and the shelf (304) are not moved along when the platform is displaced.

14. The method of claim 13, wherein in the operational state the CT table enters the opening of the annular gantry in a first direction relative to the platform, and the platform is displaced in a second direction opposite to the first direction relative to the standing surface.

## Revendications

1. Installation (100) de tomodensitométrie assistée par ordinateur pour l'examen de la partie du corps d'un grand animal, comportant :
- un appareil (102) de tomodensitomètre assisté par ordinateur (CT) ayant un portique (104) annulaire et une table (106) CT de réception de la partie du corps à examiner d'un grand animal,
- et une plateforme (110) sur laquelle l'appareil (102) CT est monté,
dans laquelle le portique (104) est immobile par rapport à la plateforme, et
dans laquelle une table CT (106) est reliée au portique et/ou à la plateforme en étant mobile horizontalement par rapport à la plateforme et par rapport au portique, de manière à ce que la table CT (106) puisse pénétrer dans une ouverture (108) du portique (104) annulaire,
**caractérisé en ce que**
l'installation (100) de tomodensitométrie assistée par ordinateur comprend un dispositif de déplacement, qui est relié à la plateforme (110) et qui est constitué pour déplacer horizontalement la plateforme (110) avec l'appareil CT (102) monté par rapport à une surface, se trouvant à l'extérieur de la plateforme (110) et où se trouve un grand animal,
dans laquelle l'installation (100) de tomodensitométrie assistée par ordinateur a un banc d'examen, qui comprend un dispositif (302) de maintien et une tablette (304) maintenue par le dispositif (302) de maintien, dans lequel le banc d'examen est disposé, de manière à ce que la tablette (304) ne soit pas déplacée en même temps, pendant un déplacement de la plateforme par le dispositif de déplacement, et
dans lequel la tablette (304) est reliée mécaniquement à la table CT, du côté tourné vers la table CT, et dans lequel la tablette CT est découplée et ainsi est mobile librement, par rapport au reste de l'appareil CT, de manière à ce que la table CT reliée à la tablette (304) reste, lorsque l'appareil CT monté sur la plateforme est déplacé ensemble avec la plateforme par le dispositif de déplacement, stationnaire par rapport au portique pendant un mouvement relatif par la liaison mécanique avec la tablette par rapport à la surface où se tient le grand animal, et de manière à ce que le dispositif (302) de maintien et la tablette (304) ne soient pas entraînés lors du déplacement de la plateforme.

2. Installation (100) de tomodensitométrie assistée par ordinateur suivant la revendication 1, qui a en outre un dispositif de levage, dans lequel le dispositif de levage est constitué pour soulever ou abaisser verticalement la plateforme (110) par rapport à la surface où se tient un grand animal, de manière à ce que l'appareil CT (102) monté sur la plateforme (110) puisse, au moyen du dispositif de levage et du dispositif de déplacement, être déplacé par rapport à la surface où se tient le grand animal, tant en étant soulevé et abaissé verticalement qu'également en étant déplacé horizontalement.

3. Installation (100) de tomodensitométrie assistée par ordinateur suivant la revendication 1 ou 2, dans laquelle le dispositif de déplacement comprend un système (114) sur rail, qui est disposé sur une plaque (112) de sol, et dans lequel la plateforme (110) est disposée avec l'appareil CT (102) monté sur le système (114) de rail, de manière à ce que la plaque (112) de sol et la plateforme (110) soient reliées entre elles de manière mobile l'une par rapport à l'autre par le système (114) de rail.

4. Installation (100) de tomodensitométrie assistée par ordinateur suivant au moins l'une des revendications 1 à 3, dans laquelle le dispositif de déplacement comprend un moteur de déplacement, qui est constitué pour mettre à disposition un entraînement de déplacement pour le déplacement de la plateforme (110) par rapport à la surface où se tient un grand animal et dans laquelle le dispositif de déplacement comprend une broche filetée et un filetage antagoniste monté déplaçable sur la broche filetée, dans laquelle le moteur de déplacement est, pour l'entraînement de la broche filetée, relié à la broche filetée et le filetage antagoniste est disposé sur la plateforme (110), de sorte que, lorsque le moteur de déplacement fonctionne, une rotation de la broche filetée se transforme en un déplacement en translation de la plateforme (110) par rapport à la surface où se tient le grand animal.

5. Installation (100) de tomodensitométrie assistée par ordinateur suivant au moins l'une des revendications 1 à 4, dans laquelle l'appareil CT comprend un entraînement CT pour l'entrée motorisée de la table CT (106) dans le portique (104), et dans laquelle un entraînement de déplacement et l'entraînement CT sont accouplés entre eux mécaniquement par un accouplement, dans laquelle l'accouplement est conformé, de manière à ce que l'entraînement CT soit directement en prise et soit transmis à l'entraînement de déplacement.

6. Installation (100) de tomodensitométrie assistée par ordinateur suivant la revendication 5, dans laquelle l'accouplement mécanique de l'entraînement CT et de l'entraînement de déplacement est conformé de manière à ce que la table CT et la plateforme (110) se déplacent en fonctionnement dans des sens contraires l'un à l'autre respectivement avec en valeur absolue la même vitesse.

7. Installation (100) de tomodensitométrie assistée par ordinateur suivant au moins l'une des revendications 2 à 6, dans laquelle la plateforme (110) est disposée avec l'appareil CT (102) sur une plaque (112) de sol et le dispositif de levage comprend une pluralité de broches (120) filetées, qui sont disposées par rapport à la plaque (112) de fond verticalement en direction de la plateforme (110) latéralement à la plaque (112) de fond et qui sont reliées respectivement à la plaque (112) de fond par un filetage (122) antagoniste monté coulissant.

8. Installation de tomodensitométrie assistée par ordinateur suivant la revendication 7, dans laquelle le dispositif de levage comprend au moins un moteur (124) de levage, qui, pour l'entraînement d'au moins l'une de la pluralité des broches (120) filetées du dispositif de levage, est relié à l'une des broches (120) filetées et est constitué pour mettre à disposition un entraînement pour le levage ou l'abaissement de la plateforme (110) par rapport à la surface où se tient un grand animal.

9. Installation de tomodensitométrie assistée par ordinateur suivant au moins l'une des revendications 2 à 8, dans laquelle la plateforme est disposée avec l'appareil CT sur une plaque de sol et le dispositif de levage comprend au moins un vérin hydraulique de pression, qui est disposé par rapport à la plaque de sol, de manière à pouvoir soulever ou abaisser la plaque de sol par rapport à la surface où se tient le grand animal, par l'alimentation en pression du vérin de pression.

10. Installation (300) de tomodensitométrie assistée par ordinateur suivant au moins l'une des revendications 1 à 9, dans laquelle l'appareil CT est un appareil CT habituel du commerce, dans lequel le portique est tourné de 180° par rapport à la table CT.

11. Installation (300) de tomodensitométrie assistée par ordinateur suivant la revendication 1, qui comprend une plaque de sol, sur laquelle la plateforme avec l'appareil CT monté est disposé, dans laquelle le dispositif (302) de maintien est monté sur la plaque de sol, de manière à ce que le dispositif (302) de maintien et la tablette (304) se déplacent conjointement avec la plaque de sol, lorsque celle-ci est soulevée ou abaissée par rapport à la surface où se tient le grand animal, par un dispositif de levage.

12. Système (200) d'examen à tomodensitomètre assisté par ordinateur comportant une installation (202) de tomodensitométrie assistée par ordinateur suivant au moins l'une des revendications 1 à 11, une surface (204) où se tient un grand animal et une fosse (206) dans le sol, dans lequel l'installation (202) de tomodensitométrie assistée est disposée dans la fosse dans le sol, de manière à ce qu'au moins une partie de l'installation (202) de tomodensitométrie assistée par ordinateur se trouve à un niveau plus bas que la surface (204) où se tient le grand animal, et dans lequel la surface (204) où se tient le grand animal se trouve à l'extérieur de la fosse (206) dans le sol au voisinage de la paroi (220) latérale de la fosse (206) dans le sol, qui est la plus près du portique (210) de l'installation (202) de tomodensitométrie assistée par ordinateur.

13. Procédé d'examen d'une partie du corps d'un grand animal par une installation de tomodensitométrie assistée par ordinateur, dans lequel l'installation de tomodensitométrie assistée par ordinateur comporte :
- un appareil de tomodensitométrie assisté par ordinateur (CT) ayant un portique annulaire et une table CT de réception d'une partie du corps à examiner d'un grand animal, et
- une plateforme sur laquelle l'appareil CT est monté, dans lequel le portique (104) est immobile par rapport à la plateforme,
dans lequel la table CT (106) est reliée au portique et/ou à la plateforme, de manière mobile horizontalement par rapport à la plateforme et au portique, de manière à ce que la table CT (106) puisse entrer dans une ouverture (108) du portique (104) annulaire,
dans lequel le procédé a les stades :
- mise en position d'un grand animal sur une surface où il se tient, qui se trouve à l'extérieur de la plateforme,
- mise d'une partie du corps à examiner sur la table CT ou sur une tablette de l'appareil CT,
- déplacement de la plateforme avec l'appareil CT par rapport à la surface où se tient le grand animal,
dans lequel le dispositif de déplacement est constitué pour, dans au moins un état de fonctionnement de la plateforme (110), être déplacé avec l'appareil CT (102) et
de manière à ce que l'appareil CT soit découplé et ainsi mobile librement par rapport au reste de l'appareil CT, de manière à ce que la table CT reliée à la tablette (304) reste, lorsque l'appareil CT monté sur la plateforme est déplacé ensemble avec la plateforme par le dispositif de déplacement, stationnaire par rapport au portique pendant un mouvement relatif, par la liaison mécanique avec la tablette par rapport à la surface où se tient le grand animal, et de manière à ce que le dispositif (302) de maintien et la tablette (304) ne soient pas entraînés lors du déplacement de la plateforme.

14. Procédé suivant la revendication 13, dans l'état de fonctionnement la table CT pénètre par rapport à la plateforme dans l'ouverture du portique annulaire suivant un premier sens et on déplace la plateforme par rapport à la surface où se tient un grand animal dans un deuxième sens, qui est contraire au premier sens.
